# EUROPÄISCHE PATENTANMELDUNG

(11) **EP 0 644 266 A1**
(43) Veröffentlichungstag der Anmeldung: **22.03.1995**
(21) Anmeldenummer: 94114051.9
(22) Anmeldetag: 07.09.1994
(51) Int. Cl.: C12Q 1/00, G01N 27/327

(54) **Arbeitselektrode für ekektrodechemisch-enzymatische Sensorsysteme**

(30) Priorität: 22.09.1993 DE 4332251
(71) Anmelder: SIEMENS AKTIENGESELLSCHAFT, D-80333 München (DE)
(72) Erfinder: Birkle, Siegfried Dr., D-91315 Höchstadt (DE); Kammermaier, Johann Dr., verstorben (DE); Schulte, Rolf, D-91056 Erlangen (DE)

(57) **Zusammenfassung**

Eine Arbeitselektrode für elektrochemisch-enzymatische Sensorsysteme weist einen metallischen Grundkörper auf, der mit einer dünnen Schicht aus amorphem wasserstoffhaltigem Kohlenstoff (a-C:H) versehen ist.

## Beschreibung

Die Erfindung betrifft eine Arbeitselektrode für elektrochemisch-enzymatische Sensorsysteme sowie ein Verfahren zur Herstellung einer derartigen Arbeitselektrode.

Zur medizintechnischen Erfassung von Stoffwechselvorgängen, beispielsweise des Glucoseumsatzes, in Körperflüssigkeiten werden Sensorsysteme eingesetzt, in denen als Kenngröße elektrochemische Potentialverschiebungen dienen, die durch enzymatische Reaktionen bewirkt werden. An die Arbeitselektrode derartiger Sensorsysteme werden folgende Anforderungen gestellt:
- elektrische Leitfähigkeit > 10⁻² Ω⁻¹·cm⁻¹
- Formvariabilität für spezielle Elektrodengeometrien
- chemisch inerter Charakter, insbesondere hinsichtlich der elektrochemisch erzeugten Umsetzungsprodukte
- Körperverträglichkeit des Elektrodenmaterials
- glatte Oberflächen, ohne Rauhigkeiten und Korngrenzen, zur Vermeidung einer Thrombosegefahr bei der Implantation.

Aus der EP-OS 0 470 290 ist ein elektrochemisch-enzymatischer Sensor zur Bestimmung von Substanzen in Körperflüssigkeiten, insbesondere von Glucose, bekannt. Dieser Sensor weist folgende Merkmale auf:
- eine Sensorelektrode aus elektrokatalytisch inaktivem Kohlenstoff
- eine Gegenelektrode
- eine Bezugselektrode
- eine vor der Sensorelektrode befindliche Enzym enthaltende Schicht und
- eine die Enzymschicht zur Körperflüssigkeit hin abdeckende und das Enzym zurückhaltende Membran aus biokompatiblem, hydrophilem, sauerstoffdurchlässigem Material.

Bei diesem Sensor dient als Material für die Sensorelektrode, die auch als Arbeitselektrode oder als Meßelektrode bezeichnet wird, Glaskohlenstoff, Pyrographit, gesputterter Kohlenstoff, gesputterter Graphit oder amorpher wasserstoffhaltiger Kohlenstoff. Vorzugsweise wird dabei Glaskohlenstoff eingesetzt, und zwar in Form einer glatten Glaskohlenstoffelektrode.

Glaskohlenstoff wird im allgemeinen durch Pyrolyse von Polyfurfurylalkohol hergestellt. Dieses Material ist - als Kohlenstoffmodifikation - chemisch genügend inert und es weist - aufgrund der amorphen Struktur - eine glatte Oberfläche auf; außerdem eignet es sich zur Implantation. In bezug auf die elektrische Leitfähigkeit erfüllt Glaskohlenstoff ebenfalls die gestellten Anforderungen. Von Nachteil ist aber, daß Glaskohlenstoff nur schwierig zu kontaktieren ist. Außerdem erfordert Glaskohlenstoff eine aufwendige Prozeßtechnik, da - bei der herstellungsbedingten Hochtemperaturpyrolyse - in dünnen Schichten leicht Risse entstehen können.

Aufgabe der Erfindung ist es, eine Arbeitselektrode für elektrochemisch-enzymatische Sensorsysteme bereitzustellen, welche den an eine derartige Elektrode gestellten Anforderungen im vollen Maße genügt.

Dies wird erfindungsgemäß dadurch erreicht, daß die Arbeitselektrode einen metallischen Grundkörper aufweist, der mit einer dünnen Schicht aus amorphem wasserstoffhaltigem Kohlenstoff (a-C:H) versehen ist.

Amorpher wasserstoffhaltiger Kohlenstoff ist eine Kohlenstoffmodifikation mit diamantähnlichen mechanischen und chemischen Eigenschaften, die Pinhole-freie, glatte Oberflächen bildet (siehe beispielsweise: "IDR - Industrie Diamanten Rundschau", Bd. 18 (1984), Seiten 249 bis 253). Mit a-C:H beschichtete Oberflächen sind in gleicher Weise wie Glaskohlenstoff gewebeverträglich und gegenüber Körperflüssigkeiten, wie Blut, inert. Ein grundlegender Unterschied zu Diamant besteht darin, daß sich der elektrische Widerstand durch die Abscheidebedingungen intrinsisch in einem weiten Bereich steuern läßt, wobei sogar mit Graphit vergleichbare Leitfähigkeiten erzielt werden können (bis zu 10⁻¹ Ω⁻¹·cm⁻¹). Die amorphe Struktur von a-C:H ergibt sich durch das Vorhandensein von sp³- und sp²-Bindungen der C-Atome, und sie wird durch chemisch gebundene H-Atome (maximaler Anteil: ca. 60 Vol.-%) bei Temperaturen bis etwa 500°C stabilisiert.

Die a-C:H-Schicht weist vorzugsweise eine Dicke < 5 µm auf. Der metallische Grundkörper besteht vorzugsweise aus Titan, daneben kommen aber auch Metalle wie Zirconium, Molybdän und Wolfram in Frage sowie hochdotiertes, d.h. elektrisch leitfähiges Silicium.

Zur Herstellung der Arbeitselektrode nach der Erfindung bzw. zur Abscheidung der dünnen a-C:H-Schicht auf dem metallischen Grundkörper dient eine Niederdruck-Plasmaabscheidung mit Hochfrequenzanregung; die Arbeitsfrequenz beträgt beispielsweise 13,56 MHz. Als Prozeßgas dienen Kohlenwasserstoffe, insbesondere CH₄, C₂H₄ und C₆H₆, vorzugsweise bei einem Arbeitsdruck zwischen 1 und 100 Pa. Die HF-Leistungsdichte im Plasma liegt vorzugsweise zwischen 0,1 und 10 W·cm⁻³. Bei ungleichen Elektrodenflächen (Verhältnis etwa zwischen 0,1 und 0,5) stellt sich eine DC-Self-bias-Spannung bis zu 1100 V ein, wobei die kleinere Elektrode die Kathode ist. Aufgrund dieser DC-Self-bias-Spannung erfolgt die a-C:H-Bildung vorwiegend als Ionenabscheideprozeß mit einer sehr hohen Energie der auf das Substrat auftreffenden CₓH_{y}-Teilchen. Dadurch ergibt sich sowohl ein hoher Anteil an diamantartigen sp³-Bindungen der C-Atome in der a-C:H-Schicht als auch - infolge einer Carbidbildung in der Grenzschicht - eine hohe Haftfestigkeit der a-C:H-Schicht am metallischen Grundkörper . Dies ist - im Gegensatz dazu - bei der CVD-Abscheidung von Pyrokohlenstoff und bei der pyrolytischen Abscheidung von Glaskohlenstoff nicht der Fall.

Die Form der Elektroden (im Abscheidungsreaktor) wird der Form der zu beschichtenden metallischen Grundkörper angepaßt. So werden beispielsweise bei ebenen Substratflächen planparallele Elektroden verschiedener Größe verwendet, bei gewölbten Grundkörpern dagegen entsprechend geformte, beispielsweise sphärisch ausgebildete Elektrodenpaare, um einen ausreichenden DC-Self-bias-Effekt zu erzielen.

Eine vorteilhafte Ausgestaltung der Erfindung besteht darin, in die a-C:H-Schicht während der Plasmaabscheidung aus einem im Abscheidungsreaktor befindlichen Target - zur Verbesserung der elektrischen Leitfähigkeit - Metallcluster einzubauen, beispielsweise aus Gold oder Titan (siehe dazu auch: "Surface and Coatings Technology", Vol. 39/40 (1989), Seiten 275 bis 284 und 285 bis 292). In diesem Fall wird die a-C:H-Abscheidung unter geänderten Bedingungen durchgeführt, d.h. das Substrat wird an der Anode positioniert. Die Kathode trägt das Target, aus dem die Metallatome durch aufprallende CₓH_{y}-Ionen oder durch Ar-Ionen abgespalten werden.

Anhand von Ausführungsbeispielen soll die Erfindung noch näher erläutert werden.

### Beispiel 1

Auf einem ebenen Titanblech mit einer Dicke von 0,25 mm, das mit einem Lösungsmittel gereinigt und entsprechend den nachfolgend beschriebenen Bedingungen einer Plasmavorbehandlung mit Argon unterzogen wurde, wird eine ca. 1,8 µm dicke a-C:H-Schicht abgeschieden. Als Prozeßgas dient Methan, der Arbeitsdruck beträgt 20 Pa. Bei einem Elektrodenflächenverhältnis von ca. 0,16 zwischen der das Substrat tragenden Elektrode und der topfförmig ausgestalteten Gegenelektrode stellt sich - bei einer Arbeitsfrequenz von 13,56 MHz und einer HF-Leistungsdichte von 0,3 W·cm⁻³ - im Plasma eine DC-Self-bias-Spannung von ca. 850 V ein, wobei die kleinere Elektrode (Fläche: 44,2 cm²) zur Kathode wird. Die erhaltene a-C:H-Schicht weist einen spezifischen elektrischen Widerstand von etwa 10⁴ Ω·cm auf. Die derart beschichtete Titanelektrode zeigt bei elektrochemischen Messungen eine sehr kleine Doppelschichtkapazität von 4,4 µF·cm⁻², was auf eine sehr glatte und dichte a-C:H-Beschichtung schließen läßt.

### Beispiel 2

Entsprechend Beispiel 1 werden bei einem Arbeitsdruck von 3,5 Pa, einer HF-Leistungsdichte im Plasma von 6,5 W·cm⁻³, einer DC-Self-bias-Spannung von ca. 1100 V und bei der Verwendung ebener Elektroden mit einem Flächenverhältnis von ca. 0,27, unter sonst gleichen Bedingungen, auf Grundkörpern aus Titan 0,4 µm dicke a-C:H-Schichten erhalten, die einen elektrischen Widerstand von lediglich 7 Ω·cm besitzen.

## Patentansprüche

1. Arbeitselektrode für elektrochemisch-enzymatische Sensorsysteme, **dadurch gekennzeichnet,** daß sie einen metallischen Grundkörper aufweist, der mit einer dünnen Schicht aus amorphem wasserstoffhaltigem Kohlenstoff (a-C:H) versehen ist.

2. Arbeitselektrode nach Anspruch 1, **dadurch gekennzeichnet,** daß die a-C:H-Schicht eine Dicke < 5 µm besitzt.

3. Arbeitselektrode nach Anspruch 1 oder 2, **dadurch gekennzeichnet,** daß der metallische Grundkörper aus Titan besteht.

4. Arbeitselektrode nach einem der Ansprüche 1 bis 3, **dadurch gekennzeichnet,** daß die a-C:H-Schicht Metallcluster enthält, insbesondere aus Gold oder Titan.

5. Verfahren zur Herstellung einer Arbeitselektrode nach einem oder mehreren der Ansprüche 1 bis 4**, dadurch gekennzeichnet,** daß auf einem metallischen Grundkörper durch Niederdruck-Hochfreguenz-Plasmaabscheidung gasförmiger Kohlenwasserstoffe eine dünne a-C:H-Schicht abgeschieden wird.

6. Verfahren nach Anspruch 5, **dadurch gekennzeichnet,** daß die Plasmaabscheidung bei einem Druck von 1 bis 100 Pa erfolgt.

7. Verfahren nach Anspruch 5 oder 6, **dadurch gekennzeichnet,** daß die Hochfrequenzleistungsdichte im Plasma 0,1 bis 10 W·cm⁻³ beträgt.

8. Verfahren nach einem der Ansprüche 5 bis 7, **dadurch gekennzeichnet,** daß die Plasmaabscheidung bei einer DC-Self-bias-Spannung bis zu 1100 V erfolgt.

9. Verfahren nach einem oder mehreren der Ansprüche 5 bis 8, **dadurch gekennzeichnet,** daß als Kohlenwasserstoff Methan vervendet wird.

10. Verfahren nach einem oder mehreren der Ansprüche 5 bis 9, **dadurch gekennzeichnet,** daß bei der Plasmaabscheidung durch Absputtern aus einem Target Metallcluster in die a-C:H-Schicht eingebaut werden.
